(19) **Europäisches Patentamt / European Patent Office / Office européen des brevets**

(11) **EP 2 428 203 A1**

(12) # EUROPEAN PATENT APPLICATION
published in accordance with Art. 153(4) EPC

(43) Date of publication:
**14.03.2012 Bulletin 2012/11**

(51) Int Cl.:
***A61K 9/107*** *(2006.01)*     ***A61K 31/475*** *(2006.01)*
***A61P 35/00*** *(2006.01)*

(21) Application number: **09844293.2**

(22) Date of filing: **08.12.2009**

(86) International application number:
**PCT/CN2009/075409**

(87) International publication number:
**WO 2010/127541 (11.11.2010 Gazette 2010/45)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **06.05.2009 CN 200910050689**

(71) Applicants:
 • **Jiangsu Hengrui Medicine Co., Ltd.**
 **Lianyungang, Jiangsu 222002 (CN)**
 • **Shanghai Hengrui Pharmaceutical Co. Ltd.**
 **Minhang District**
 **Shanghai 200-245 (CN)**

(72) Inventors:
 • **TONG, Xinyong**
 **Shanghai 200245 (CN)**
 • **WANG, Haifeng**
 **Shanghai 200245 (CN)**
 • **CUI, Shuangjin**
 **Shanghai 200245 (CN)**
 • **YU, Li**
 **Shanghai 200245 (CN)**

(74) Representative: **Crowhurst, Charlotte Waveney et al**
 **Potter Clarkson LLP**
 **Park View House**
 **58 The Ropewalk**
 **Nottingham**
 **NG1 5DD (GB)**

(54) **A NANO-EMULSION INJECTION OF VINCA ALKALOIDS AND THE PREPARATION METHOD THEREOF**

(57)     A nano-emulsion injection of Vinca alkaloids and its preparation method are disclosed. The injection is an oil-in-water emulsion injection comprising Vinca alkaloids or their salts, injectable oil, surfactant(s) and injectable water, wherein the average diameter of the droplets of the emulsion is less than 100 nm and the pH of the emulsion is 7-9. The preparation method comprises the steps of preparing the oil phase and the aqueous phase respectively, homogeneously mixing the oil phase and the aqueous phase with high speed, adding the active ingredient, adjusting the pH to 7-9, adding water to constant volume, and homogenizing the emulsion till the average diameter of the droplets being less than 100 nm. The alternative method comprises the steps of homogeneously mixing the oil phase and the aqueous phase, homogenizing the obtained emulsion under high pressure till the average diameter of the droplets being less than 100 nm, adding the active ingredient, adjusting the pH to 7-9, stirring, and adding water to constant volume.

**EP 2 428 203 A1**

Description

FIELD OF THE INVENTION

The present disclosure relates to a nano-emulsion injection and the preparation method thereof, especially relates to a nano-emulsion injection of vinca alkaloids and the preparation method thereof.

BACKGROUND OF THE INVENTION

Vinorelbine is a class of semisynthetic vinca alkaloids agent. As the representative drug of the third-generation vinca alkaloids, it has strong antitumor activity, definite effect and lower neurotoxicity than other vinca alkaloids agents such as vinblastine. Vinorelbine is widely used in the clinical treatment and in the combination therapy. It's more effective in the treatment of non-small-cell carcinoma and breast cancer, and it is convenient for drug administration, which is a mature drug, has good market and sales.

The present marketed products are Injection of Vinorelbine Tartrate (Navelbine®) and soft capsule in China, there's no other dosage forms launched yet.

As the present injection products in the market are acidic hypertonic solutions, they cause vascular stimulation seriously, especially to the vein, and result in phlebitis, which is presented as cutaneous pigmentation, vascular pain or vascular harden etc. Studies have shown that the said side effects occur in about one third of the patients and 5% of those have serious reactions. Therefore, rapid intravenous injections are required within 30 minutes in clinical use with dexamethasone IVP before and after chemotherapy and vascular being flushed with plenty of saline water, which brings much inconvenience in clinical application.

Some patent applications of vinorelbine emulsion were disclosed in China. Chinese Patent Application Publication No. CN1859898A disclosed sub-micron size oil-in-water emulsion of vinca alkaloid drug, in which the average diameter of oil droplets was more than 115 nm. This patent application also disclosed only when the said vinca alkaloid emulsion has an acidic pH (e.g. pH3-5) and high concentration stabilizer (the charge ratio between stabilizer and drug ranges from 1:1 to 10:1, and the average amount of the stabilizer is 1.5-7.5% of emulsion), the emulsion is stable and vinca alkaloid can be partitioned well in oil phase. The inventor of this application intended to encapsulate Vinorelbine tartrate in the internal phase of oil-in-water emulsion droplets to prevent the direct contact of drug with venous endothelium tissue so that the side effects of vascular stimulation were reduced or avoided. However, this emulsion still has vascular stimulation for its strong acidity. If the pH is increased to alkalinity to reduce vascular stimulation, the emulsion will be not stable any more.

Since vinca alkaloids compounds are highly water-soluble, it is critical to make it encapsulated in the oil droplets maximally during the preparation of the oil-in-water emulsion. Chinese patent applications (CN1771954A, CN1679576A and CN1634058A) disclosed some kinds of vinca alkaloids emulsions, all of which made no special control to the particle size, so the particle size is as large as sub-micron size (i.e., the average diameter is more than 100 nm), that is, the diameter of the resulting emulsion is larger. In these patent applications, large amounts of excipients such as oil solubilizer, cosolvent or high concentration stabilizer with similar function were employed to encapsulate drug into oil phase, so the formulation is complex. For example, 10-hydroxy-2-decenoic acid or/and sorbitan fatty acid ester were used as oil solubilizer to increase lipophilicity of vinorelbine in oil phase, or ethanol and 1, 2-propylene glycol were used as cosolvent to dissolve intramuscularly dosage vinca alkaloids into the oil phase of the emulsion. Although these emulsions use many complex excipients, satisfied encapsulation efficiency cannot be achieved and a lot of safety risks will exist. Meanwhile, it was proved that the emulsions prepared by these methods were not stable during storage, because drug can transfer from oil phase to the aqueous phase easily with the results that encapsulation efficiency decreases.

Since the droplet size is required to be controlled within micron size or even smaller in the use of vinca alkaloids fat emulsion injection, conventional emulsion is prepared by dispersing vinca alkaloids drug firstly and then homogenizing the emulsion. During the course of homogenization, higher pressure and temperature will cause adverse impact on drug stabilization.

With regard to the above problems, it is important to provide a new kind of vinca alkaloids emulsion with high encapsulation efficiency, high human tolerability and high storage stability.

DESCRIPTION OF THE INVENTION

One objective of the present invention is to overcome the clinical stimulation of current vinca alkaloids injections, decrease toxicity, and provide a safe, stable vinca alkaloids emulsion injection with high encapsulation efficiency. As to the deficiency of the prior art, another objective of the present invention is to provide a new preparation method of emulsion to resolve the problems that active ingredients dissolve slowly or hardly in oil phase during the emulsion preparation process.

The present invention provides a stable oil-in-water emulsion injection comprising vinca alkaloids or pharmaceutically acceptable salts, injectable oil, surfactants and water. It has been proved that on the condition that pH is 7-9, the average diameter is less than 100 nm, the drug encapsulation efficiency could be increased, the drug could not easily leak from oil phase, and should be much more suitable for use in human body, and the emulsion could be prepared more gently.

The present invention provides a nano-emulsion injection of vinca alkaloids, **characterized in that** the said injection is an oil-in-water emulsion injection comprising vinca alkaloids or pharmaceutically acceptable salts, injectable oil, surfactants and injectable water, wherein the said emulsion have droplets with an average diameter less than 100 nm and the pH value is 7-9.

The vinca alkaloids according to this invention are extractive, synthetic or semisynthetic, wherein the said salts are prepared by the reaction between vinca alkaloids and pharmaceutically acceptable acid. Vinca alkaloids include, but not limited to, vinorelbine, vinblastine, vincristine, vindesine and vinrosidine. The salts of vinca alkaloids include, but not limited to, tartrate, maleate, lactate, malate, hydrochloride, phosphate and sulfate, preferably vinorelbine or vinorelbine tartrate.

The vinca alkaloids are presented in the said nano-emulsion injection in the range of 0.05-5% (w/v), more preferably 0.05-1.0 % (w/v).

The injectable oil according to this invention is selected from the group consisting of one or more mineral oil, plant oil, animal oil and synthetic oil. The said plant oil is selected from the group consisting of soybean oil, safflower oil, corn oil, coconut oil, castor oil, brucea javanica oil, palm oil, medium chain fatty acid triglycerides, peanut oil, cottonseed oil and a mixture thereof. The said animal oil is selected from the group consisting of fish oil, sperm oil and a mixture thereof, preferably soybean oil, medium chain fatty acid triglycerides and a mixture thereof.

The injectable oil is presented in the said nano-emulsion injection in the range of 2-30% (w/v), more preferably 5-20% (w/v).

The surfactants according to this invention are selected from the group consisting of phospholipids, nonionic surfactant and a mixture thereof. The said phospholipids are selected from the group consisting of lecithin, soybean lecithin and a mixture thereof, preferably egg lecithin. The said nonionic surfactant is selected from the group consisting of polyoxyethylene nonionic surfactant and polyethylene glycol nonionic surfactant. The said polyoxyethylene surfactants are preferably selected from the group consisting of polyoxyethylene castor oil, poly (ethylene oxide) hydrogen castor oil, tween 20, tween 40, tween 60, tween 80, tween 85, poloxamer188 and a mixture thereof. The said polyethylene glycol nonionic surfactant is preferably selected from the group consisting of polyethylene glycol stearate 15, polyethylene glycol- vitamin E succinate and a mixture thereof. The said nonionic surfactants are more preferably selected from the group consisting of poloxamer188 and polyethylene glycol stearate 15. A combined use of phospholipids and nonionic surfactant is preferable to decrease emulsion particle size effectively and increase emulsion storage stability. The preferable combined surfactants are lecithin and poloxamer 188, or lecithin and polyethylene glycol stearate 15, and more preferably from: egg lecithin and poloxamer 188, or egg lecithin and polyethylene glycol stearate 15.

The surfactant is presented in the said nano-emulsion injection in the range of 1-20% (w/v), more preferably 2-10 % (w/v).

Further, the present invention provides a more preferred nano-emulsion injection of vinca alkaloids comprising:

0.05-1.0 w/v % of vinorelbine or the tartrate form based upon the said nano-emulsion injection;
5-20 w/v % of soybean oil or medium chain fatty acid triglycerides or a mixture thereof based upon the said nano-emulsion injection;
2-10 w/v % of combined surfactants based upon the said nano-emulsion injection, whereas the said combined surfactants are lecithin and poloxamer 188, or lecithin and polyethylene glycol stearate 15, more preferably egg lecithin and poloxamer 188, or egg lecithin and polyethylene glycol stearate 15.

The nano-emulsion injection according to this invention may further comprise a metal chelator, wherein the metal chelator is EDTA, EDTA disodium salt, EDTA dicalcium salt and a mixture thereof, and preferably EDTA disodium salt. The metal chelator is presented in the said injectable nano-emulsion in the range of 0-0.5% (w/v).

The nano-emulsion injection according to this invention may further comprise an antioxidant including water-soluble antioxidant and oil-soluble antioxidant. The said water-soluble antioxidant is sodium sulfite, sodium hydrogensulfite, sodium metabisulfite, ascorbic acid, sodium ascorbate, L-cysteine or a mixture thereof, preferably sodium sulfite. The said oil-soluble antioxidant is vitamin E. The antioxidant is presented in the said nano-emulsion injection in the range of 0-1% (w/v).

The addition of metal chelator and antioxidant can increase the chemical stability of emulsion.

The nano-emulsion injection according to this invention may further comprise an osmotic pressure regulator. The said osmotic pressure regulator is glycerin, sorbitol, mannitol, glucose, sodium chloride or a mixture thereof, preferably glycerin. The osmotic pressure regulator is presented in the said nano-emulsion injection in the range of 0-5% (w/v).

The nano-emulsion injection according to this invention may further comprise a cosurfactant. The said cosurfactant has the function of surface activity and can adjust the charge of the emulsion system, thus increasing the repulsive force among emulsion droplets and enhancing the emulsion stability. The said cosurfactant is oleic acid, sodium oleate, cholic acid, sodium cholate, deoxycholic acid, deoxysodium cholate or a mixture thereof, preferably sodium oleate. The cosurfactant is presented in the said nano-emulsion injection in the range of 0-1.5% (w/v).

The said emulsion may further comprise lowly concentrated ingredient which can enhance the lipophilicity of vinca alkaloids in oil phase, but preferably, the said ingredient is absent in the emulsion.

The present invention also provides methods to prepare the said vinca alkaloids nano-emulsion injection.

Method 1:

The method comprises the following steps of:

Preparing the oil phase and the aqueous phase respectively; homogeneously mixing the oil phase and the aqueous phase to obtain coarse emulsion; adding vinca alkaloids or the salts thereof into the coarse emulsion then adjusting the pH value to 7-9, further adding water into the container to the constant volume, then homogenizing the emulsion with high pressure homogenizer till the average diameter of the droplets being less than 100nm.

For example, under the protection of inert gas atmosphere, stirring injectable oil and the other optionally oil-soluble excipients of the formulation homogeneously to obtain oil phase; adding surfactants and the other optionally water-soluble excipients of the formulation into an appropriate amount of injectable water, then homogeneously stirring them to obtain the aqueous phase;

Homogeneously mixing the oil phase and the aqueous phase with high speed to obtain coarse emulsion;

Adding vinca alkaloids or the salts thereof into the coarse emulsion;

Adjusting the pH value to 7-9, further add water to the constant volume, then homogenizing the emulsion with high pressure homogenizer till the average diameter of the droplets being less than 100 nm.

Method 2:

The average diameter of the emulsion according to this invention is controlled to be less than 100 nm and the pH value is 7-9. In that case, drug is easy to dissolve into oil phase when it is gently stirred. Besides Method 1, the said products may be prepared by a gentler preparation comprising the following steps of:

Preparing the oil phase and the aqueous phase respectively; homogeneously mixing the oil phase and the aqueous phase, and homogenizing the emulsion with high pressure homogenizer to obtain a blank emulsion with an average diameter less than 100 nm; adding vinca alkaloids or the salts thereof into the blank emulsion; then adjusting the pH value to 7-9, stirring the blank emulsion thoroughly, further adding water to the constant volume.

For example, under the protection of inert gas atmosphere, homogeneously stirring injectable oil and the other optionally oil-soluble excipients of the formulation to obtain oil phase, adding surfactants and the other optionally water-soluble excipients of the formulation into an appropriate amount of injection water, then stirring them homogeneously to obtain the aqueous phase;

Homogeneously mixing the said oil phase and the aqueous phase, and homogenizing the emulsion with high pressure homogenizer to obtain a blank emulsion with an average diameter less than 100 nm;

Adding vinca alkaloids or the salts thereof into the blank emulsion;

Adjusting the pH value to 7-9, further adding water to the constant volume, and homogeneously stirring it.

In the two preparation methods mentioned above, the surfactants can not only be dispersed into the aqueous phase, but also can be dissolved or dispersed into the oil phase, such as dissolving egg lecithin into the oil phase.

In Method 2, the average diameter of blank nano-emulsion is controlled to be less than 100 nm before drug is added. The small average diameter may cause highly dispersed emulsion droplets. After drug has been added into the system, because of great difference of solubility between the oil phase and the aqueous phase, and the surface area of the highly dispersed emulsion droplets is big enough, vinca alkaloids can disperse into oil-water interfacial film quickly, and then enter into the oil phase. Nano-emulsion containing drug was not prepared by homogenizing emulsion containing drug intensively, but by adding drug into preformed blank nano-emulsion to avoid the adverse impact on the drug stability during the strong homogenizing process in Method 2.

Therefore, Method 2 is preferable.

Comparing with the prior art, the present invention, by controlling the average diameter of emulsion droplets and pH value, achieves the following purposes:

1. To reduce vascular stimulation of the product

The nano-emulsion appears to be transparent or semitransparent with a little opalescence, and has low viscosity. It can reduce vascular stimulation in intravenous injection, and the pH value is more acceptable to human body.

2. To decease drug toxicity

Nausea and vomiting is the most common adverse effects of chemotherapy drugs, even aphagia in some patients. As an anticancer drug, vinorelbine also has these adverse effects. The product according to this invention has similar components with fat emulsion, so it can provide necessary energy for human body and maintain the structure of normal cells to improve the patients' clinical tolerance.

The diameter of the product according to this invention is less than 100 nm. It can reduce toxicity much more than common emulsions.

3. Simple process and high encapsulation efficiency

Blank nano-emulsion is preferably prepared by high pressure homogenization according to this invention. Because the average diameter of droplets is less than 100 nm, the emulsion droplets have a high degree of dispersity. After drug has been added into the system, because of great difference of solubility between the oil phase and the aqueous phase, and the surface area of the highly dispersed emulsion droplets is big enough, vinca alkaloids can disperse into oil-water interfacial film quickly, and then enter into the oil phase. So encapsulation efficiency of the product was improved. In the present invention, drug was dissolved into oil phase under simple stirring to avoid the adverse impact on the drug stability during the strong homogenizing process.

4. High stability

The diameter of nano-emulsion droplets is homogeneously between 10 and 100 nm. The system is stable, and it can be long-term stored and would not be layered after centrifugation. Comparing with the common emulsion, it is much more stable and drug cannot be easily leak from oil phase.

5. Simple formulation

The drug of the formulation according to this invention can be encapsulated into oil phase easily by using simple excipients. Therefore the present emulsion may comprise no or less oil solubilizer, cosolvent or stabilizer with similar function to retain vinca alkaloids in the oil phase to simplify the formulation.

Compared vinorelbine nano-emulsion injection according to this invention with other technology in the prior art, vinorelbine is dispersed into the oil-water interfacial film by simple formulation and preparation process, thus the drug concentration is improved and stimulation is reduce. Otherwise, the drug can be delivered in vivo in the form of nanospheres to alter drug distribution in vivo, reach highly targeting, control drug release rate to some extent, increase drug solubility and absorption rate, and improve drug potency and decreasing toxicity.

BRIEF DESCRIPTION OF THE DRAWINGS

Fig.1 shows size distribution of vinca alkaloids nano-emulsion injection according to the present invention

Fig.2 shows morphology of vinca alkaloids nano-emulsion injection according to the present invention

Fig.3 shows stimulation test result of vinca alkaloids nano-emulsion injection according to the present invention

**PREFERRED EMBODIMENTS**

The following examples are intended to illustrate the present invention.

Chromatographic conditions for determining the content of drug and related substances: the instrument has a C18 column, and the mobile phase is 0.2% sodium 1-dodecanesulfonate methanol solution -50 mM $NaH_2PO_4$ buffer solution (phosphoric acid adjusted pH value to 4.20) (61:39) with a flow rate of 1.0ml/min and detective wavelength of 267 nm and column temperature of 40°C.

Method for determination of encapsulation efficiency:

Dilute appropriate amount of this product with water, and then ultrafilter it. Discard the initial filtrate; reserve the subsequent filtrate as the sample solution. Pipette 20µL solution each of the sample and the control into liquid chromatography, record the chromatogram map. W is the drug content of the aqueous phase of the emulsion calculated by external standardization method. $W_0$ is the total amount of drug in this product by a content determination method. The encapsulation efficiency is calculated by the follow equation:

$$\text{Encapsulation Efficiency} = \frac{W_0 - W}{W_0} \times 100\%$$

EXAMPLE 1

|  |  |
|---|---|
| vinorelbine tartrate | 0.2% |
| soybean oil | 7.5% |
| medium chain oil | 7.5% |
| egg lecithin | 5% |
| polyethylene glycol stearate 15 | 4% |
| glycerin | 2.5% |
| sodium oleate | 0.1 % |
| vitamin E | 0.05% |
| sodium sulfite | 0.05% |
| EDTA-2Na | 0.01% |
| injectable water | up to 100% |

Under the protection of inert gas atmosphere, injectable soybean oil 75g, medium chain oil 75g and vitamin E 0.5g were homogeneously mixed to obtain the oil phase. Egg lecithin 50g, sodium sulfite 0.5g, sodium oleate 1g, glycerin 25g, EDTA-2Na 0.1g and polyethylene glycol stearate 15 40g were added into 700 ml injectable water and homogeneously stirred to obtain the aqueous phase. Under high-speed stirring, the oil phase and the aqueous phase were homogeneously mixed and homogenized with high pressure homogenizer to obtain a blank emulsion with an average diameter less than 100 nm. The resulting emulsion was added with vinorelbine tartrate 2g, adjusted pH value to 8.0, added with injectable water to the constant volume of 1000 ml and homogeneously stirred. The emulsion was filled under the protection of nitrogen and the container was sealed.

EXAMPLE 2

The formulation was prepared under the same conditions described in the example 1, except that pH value was adjusted to 7.0.

EXAMPLE 3

The formulation was as same as the example 1, except that pH value was adjusted to 6.5 during the process.

EXAMPLE 4

The formulation was as same as the example 1, except that pH value was adjusted to 4.5 during the process.

EXAMPLE 5

The formulation was as same as the example 1, except that pH value was adjusted to 9.0 during the process.

**EXAMPLE 6**

The formulation was as same as the example 1.

Preparation procedure: Under the protection of inert gas atmosphere, injectable soybean oil 75g, medium chain oil 75g and VE 0.5g were homogeneously stirred to obtain the oil phase. Egg lecithin 50g, sodium sulfite 0.5g, sodium oleate 1g, glycerin 25g, EDTA-2Na 0.1g and polyethylene glycol stearate 15 40 g were added into 700 ml injectable water and stirred homogeneously to obtain the aqueous phase. Under high-speed stirring, the oil phase and the aqueous phase were homogeneously mixed and homogenized with high pressure homogenizer to obtain a coarse emulsion. The resulting coarse emulsion was added with vinorelbine tartrate 2g, adjusted to pH value 8.0, added with injectable water to the constant volume of 1000 ml, homogeneously stirred and homogenized with high pressure homogenizer for the second time. The average diameter was detected as less than 100 nm. The emulsion was filled under the protection of nitrogen and the container was sealed.

The encapsulation efficiencies of examples 1-6 were calculated by the said equation and shown as follows:

Table 1: The encapsulation efficiencies of examples 1-6

|  | encapsulation efficiency(%) |
|---|---|
| Example 1 | 98.7 |
| Example 2 | 87.3 |
| Example 3 | 74.9 |
| Example 4 | 23.5 |
| Example 5 | 99.1 |
| Example 6 | 98.9 |

It was shown from examples 1-5 that the pH value of emulsion influences the encapsulation efficiency obviously. An acidic pH value causes low encapsulation efficiency. When the pH value was 4.5, the encapsulation efficiency was even less than 50%. As the rising of the pH value, the encapsulation efficiency increase obviously. And when the pH value comes near to neutral or even to alkaline, the encapsulation efficiency gets to more than 80%.

Based on the results of examples 1 and 6, it was shown that there's no significant difference between encapsulation efficiencies obtained from the following two methods: one method was to prepare blank nano-emulsion first, then add drug into the emulsion, stir the emulsion and obtain the final product; the other routine method described in example 6 was to add drug into the emulsion, homogenize the emulsion with high pressure homogenizer for the second time and obtain the final product.

**EXAMPLE 7**

The formulation was as same as the example 1.

Preparation procedure: Under the protection of inert gas atmosphere, injectable soybean oil 75g, medium chain oil 75g and VE 0.5g were mixed homogeneously to obtain the oil phase. Egg lecithin 50g, sodium sulfite 0.5g, sodium oleate 1g, glycerin 25g, EDTA-2Na 0.1g and polyethylene glycol stearate 15 40g were added into 700ml injectable water and stirred homogeneously to obtain the aqueous phase. Under high-speed stirring, the oil phase and aqueous phase were stirred homogeneously and homogenized with high pressure homogenizer to a obtain blank emulsion. The average diameter was detected as less than 200 nm. The resulting emulsion was added with vinorelbine tartrate 2g, adjusted pH value to 8.0, added with water to the constant volume of 1000 ml and stirred homogeneously. The emulsion was filled under the protection of nitrogen, and the container was sealed.

Particle size andencapsulation efficiencies of examples 1 and 7 were determined, the results were shown as follows:

Table 2 particle size and encapsulation efficiencies of examples 1 and 7

|  | Particle size of blank emulsion (nm) | Particle size of final product (nm) | Encapsulation efficiency (%) |
|---|---|---|---|
| Example 1 | 52 | 51 | 98.7 |
| Example 7 | 144 | 146 | 94.5 |

The results showed that under the condition of adopting the same formulation, the encapsulation efficiency of drug-loaded product increased when the particle size of blank emulsion was less than 100 nm.

**EXAMPLE 8**

| | |
|---|---|
| vinorelbine tartrate | 0.2% |
| soybean oil | 5.0% |
| medium chain oil | 5.0% |
| egg lecithin | 3% |
| poloxamer188 | 3% |
| glycerin | 2.5% |
| sodium oleate | 0.15% |
| vitamin E | 0.05% |
| sodium sulfite | 0.2% |
| injectable water | up to 100% |

Under the protection of inert gas atmosphere, injectable soybean oil 50g, medium chain oil 50g and vitamin E 0.5g were homogeneously stirred to obtain the oil phase. Egg lecithin 30g, poloxamer188 30g, sodium sulfite 2g, sodium oleate 1.5g and glycerin 25g were added into 700ml injectable water and homogeneously stirred to obtain the aqueous phase. Under high-speed stirring, the oil phase and the aqueous phase were homogeneously mixed and homogenized with high pressure homogenizer to obtain a blank emulsion with an average diameter less than 100 nm. The resulting emulsion was added with vinorelbine tartrate 2g, adjusted pH value to 8.0, added with injectable water to the constant volume of 1000 ml and homogeneously stirred. The emulsion was filled under the protection of nitrogen, and the container was sealed.

**EXAMPLE 9**

| | |
|---|---|
| vinorelbine tartrate | 0.2% |
| soybean oil | 5.0% |
| medium chain oil | 5.0% |
| egg lecithin | 8% |
| glycerin | 2.5% |
| sodium oleate | 0.05% |
| injectable water | up to 100% |

Under the protection of inert gas atmosphere, injectable soybean oil 50g and medium chain oil 50g were homogeneously stirred to obtain the oil phase. Egg lecithin 80g, sodium oleate 0.5g and glycerin 25g were into 700 ml injectable water and homogeneously stirred to obtain the aqueous phase. Under high-speed stirring, the oil phase and the aqueous phase were homogeneously mixed and homogenized with high pressure homogenizer to obtain a coarse emulsion. The resulting emulsion was added with vinorelbine tartrate 2g, adjusted pH value to 8.0, added with injectable water to the constant volume of 1000 ml, and stirred homogeneously, homogenized the emulsion with high pressure homogenizer for the second time. The average diameter was detected as less than 100 nm. The emulsion was filled under the protection of nitrogen, and the container was sealed.

**EXAMPLE 10**

| | |
|---|---|
| vinorelbine tartrate | 0.2% |
| medium chain oil | 10% |
| egg lecithin | 2% |

(continued)

| | |
|---|---|
| polyethylene glycol stearate 15 | 2% |
| glycerin | 2.5% |
| sodium oleate | 0.15% |
| vitamin E | 0.05% |
| EDTA-2Na | 0.02% |
| injectable water | up to 100% |

Under the protection of inert gas atmosphere, injectable medium chain oil 100g and vitamin E 0.5g homogeneously were stirred to obtain the oil phase. Egg lecithin 20g, polyethylene glycol stearate 15 20g, EDTA-2Na 0.2g, sodium oleate 1.5g and glycerin 25g were added into 700 ml injectable water and homogeneously stirred to obtain the aqueous phase. Under high-speed stirring, the oil phase and the aqueous phase were homogeneously mixed and homogenized with high pressure homogenizer to obtain a coarse emulsion. The resulting emulsion was added with vinorelbine tartrate 2g, adjusted pH value to 8.0, added with injectable water to the constant volume of 1000 ml, homogeneously stirred and homogenized with high pressure homogenizer for the second time. The average diameter was detected as less than 100 nm. The emulsion was filled under the protection of nitrogen, and the container was sealed.
Examples 1, 8-10 were determined according to Chromatographic conditions for determining the content of drug and related substances, and the results were shown in tables 3 and 4:

Table 3 Determination results before accelerating test

| | Particle size (nm) | Content (%) | Related substance (%) |
|---|---|---|---|
| Example 1 | 51 | 98.7 | 0.32 |
| Example 8 | 94 | 97.6 | 0.45 |
| Example 9 | 89 | 99.2 | 0.58 |
| Example 10 | 87 | 98.9 | 0.51 |

Table 4 Determination results of accelerating test for one month (25°C)

| | Particle size (nm) | Content (%) | Related substance (%) |
|---|---|---|---|
| Example 1 | 52 | 98.6 | 0.63 |
| Example 8 | 102 | 97.4 | 0.79 |
| Example 9 | 113 | 93.8 | 6.27 |
| Example 10 | 98 | 96.3 | 1.64 |

The results of the above four examples showed that: after accelerating test for one month, various changes occurred in particle size, content and related substance of different formulations, wherein the stability of examples 1, 8 and 10 was comparatively high. The reason was that the combined surfactants could increase emulsion storage stability, so that particle size of the emulsion changes little under the accelerating test condition. Antioxidant and metal chelator could increase the chemical stability of emulsion.

**EXAMPLE 11**

| | |
|---|---|
| vinorelbine tartrate | 0.05% |
| soybean oil | 5% |
| egg lecithin | 5% |
| glucose | 5% |
| sodium oleate | 0.1% |
| injectable water | up to 100% |

Under the protection of inert gas atmosphere, injectable soybean oil 50g and egg lecithin 50g were homogeneously mixed to obtain the oil phase. Glucose 50g and sodium oleate 1g were added into 600 ml injectable water and homogeneously stirred to obtain the aqueous phase. Under high-speed stirring, the oil phase and the aqueous phase were mixed homogeneously and homogenized with high pressure homogenizer to obtain a blank emulsion with an average diameter less than 100 nm. The resulting emulsion was added with vinorelbine tartrate 0.5g, adjusted pH value to 7.5, added with injectable water to the constant volume of 1000 ml and homogeneously stirred. The emulsion was filled under the protection of nitrogen, and the container was sealed.

**EXAMPLE 12**

| | |
|---|---|
| vinorelbine tartrate | 0.5% |
| soybean oil | 10% |
| soybean lecithin | 10% |
| glycerin | 2.25% |
| sodium oleate | 0.3% |
| EDTA-2Na | 0.05% |
| injectable water | up to 100% |

Under the protection of inert gas atmosphere, injectable soybean oil 100g was homogeneously stirred to obtain the oil phase. Soybean lecithin 100g, glycerin 22.5g, sodium oleate 3g and EDTA-2Na 0.5g were added into 600 ml injectable water and homogeneously stirred to obtain the aqueous phase. Under high-speed stirring, the oil phase and the aqueous phase were homogeneously mixed and homogenized with high pressure homogenizer to obtain a coarse emulsion. The resulting emulsion was added with vinorelbine tartrate 5g, adjusted pH value to 8.5, added with injectable water to the constant volume of 1000 ml, homogenized with high pressure homogenizer until the average diameter was less than 100 nm. The emulsion was filled under the protection of nitrogen, and the container was sealed.

**EXAMPLE 13**

| | |
|---|---|
| vinorelbine tartrate | 1.0% |
| soybean oil | 10% |
| egg lecithin | 3% |
| polyethylene glycol- vitamin E succinate | 3% |
| glycerin | 2.5% |
| sodium oleate | 0.3% |
| EDTA-2Na | 0.01% |
| ascorbic acid | 0.2% |
| injectable water | up to 100% |

Under the protection of inert gas atmosphere, injectable soybean oil 100g and egg lecithin 30g were homogeneously mixed to obtain the oil phase. Polyethylene glycol- vitamin E succinate 30g, glycerin 25g, sodium oleate 3g, EDTA-2Na 0.1g and ascorbic acid 2g were added into 800 ml injectable water and homogeneously stirred to obtain the aqueous phase. Under high-speed stirring, the oil phase and the aqueous phase were homogeneously mixed and homogenized with high pressure homogenizer to obtain a blank emulsion with an average diameter less than 100 nm. The resulting emulsion was added with vinorelbine tartrate 10g, adjusted pH value to 9.0, added with injectable water to the constant volume of 1000 ml and homogeneously stirred. The emulsion was filled under the protection of nitrogen, and the container was sealed.

**EXAMPLE 14**

| | |
|---|---|
| vinorelbine | 0.2% |
| medium chain oil | 10% |
| egg lecithin | 4% |
| tween 80 | 0.5% |
| glycerin | 2.25% |
| sodium oleate | 0.1% |
| sodium sulfite | 0.2% |
| vitamin E | 0.05% |
| injectable water | up to 100% |

Under the protection of inert gas atmosphere, injectable medium chain oil 100g and vitamin E 0.5g homogeneously were mixed to obtain the oil phase and preheated to 70°C. Tween 80 5g, sodium sulfite 2g, glycerin 22.5g, sodium oleate 1g and egg lecithin 40g were added into 650ml injectable water, homogeneously stirred to obtain the aqueous phase, and preheated to 70°C. Under high-speed stirring, the oil phase and the aqueous phase were homogeneously mixed and homogenized with high pressure homogenizer to obtain a coarse emulsion. The resulting emulsion was added with vinorelbine 2g, adjusted pH value to 7.0, added with injectable water to the constant volume of 1000 ml, and homogenized with high pressure homogenizer for the second time. The average diameter was detected as less than 100 nm. The emulsion was filled under the protection of nitrogen, and the container was sealed.

**EXAMPLE 15**

| | |
|---|---|
| vinorelbine | 0.5% |
| medium chain oil | 5% |
| egg lecithin | 4% |
| polyoxyethylene castor oil | 3% |
| glycerin | 2.25% |
| sodium oleate | 0.3% |
| EDTA-2Na | 0.01% |
| vitamin E | 0.05% |
| ascorbic acid | 0.1% |
| injectable water | up to 100% |

Under the protection of inert gas atmosphere, injectable medium chain oil 50g and vitamin E 0.5g were homogeneously mixed to obtain the oil phase. Egg lecithin 40g, polyoxyethylene castor oil 30g, glycerin 22.5g, sodium oleate 3g EDTA-2Na 0.1g and ascorbic acid 1g were added into 600 ml injectable water and homogeneously stirred to obtain the aqueous phase.

Under high-speed stirring, the oil phase and the aqueous phase were homogeneously mixed and homogenized with high pressure homogenizer to obtain a coarse emulsion. The resulting emulsion was added with vinorelbine 5g, adjusted pH value to 7.5, added with injectable water to the constant volume of 1000 ml, homogeneously stirred, and homogenized the liquid with high pressure homogenizer for the second time. The average diameter was detected as less than 100 nm. The emulsion was filled under the protection of nitrogen, and the container was sealed.

**EXAMPLE 16**

| | |
|---|---|
| vinorelbine Tartrate | 5% |
| soybean oil | 10% |
| medium chain fatty acid triglyceride oil | 5% |
| egg lecithin | 12% |
| poloxamer188 | 6% |

(continued)

| | |
|---|---|
| glycerin | 2.25% |
| sodium oleate | 0.15% |
| vitamin E | 0.05% |
| injectable water | up to 100% |

Under the protection of inert gas atmosphere, injectable soybean oil 100g, medium chain oil 50g and vitamin E 0.5g homogeneously were mixed to obtain the oil phase. Egg lecithin 120g, glycerin 22.5g, sodium oleate 1.5g and poloxamer188 60g were added into 650 ml injectable water, homogeneously stirred to obtain the aqueous phase, and preheated to 80°C. Under high-speed stirring, the oil phase and the aqueous phase were homogeneously mixed and homogenized with high pressure homogenizer to obtain a coarse emulsion. The resulting emulsion was added with vinorelbine tartrate 50g, adjusted pH value to 9.0, added injectable water to the constant volume of 1000ml, homogeneously stirred, and homogenized with high pressure homogenizer for the second time. The average diameter was detected as less than 100 nm. The emulsion was filled under the protection of nitrogen, and the container was sealed.

**EXAMPLE 17**

| | |
|---|---|
| vincristine sulfate | 2% |
| medium chain oil | 20% |
| soybean lecithin | 10% |
| polyethylene glycol stearate 15 | 5% |
| glycerin | 2.5% |
| sodium oleate | 0.2% |
| vitamin E | 0.05% |
| injectable water | up to 100% |

Under the protection of inert gas atmosphere, injectable medium chain oil 200g and vitamin E 0.5g were homogeneously mixed to obtain the oil phase and preheated to 60°C. Soybean lecithin 100g, glycerin 25g, sodium oleate 2g and polyethylene glycol stearate 15 50g were added into 700 ml injectable water, homogeneously stirred to obtain the aqueous phase and preheated to 60°C. Under high-speed stirring, the oil phase and the aqueous phase were homogeneously mixed, and homogenized with high pressure homogenizer to obtain a coarse emulsion. The resulting emulsion was added vincristine sulfate 20g, adjusted pH value to 8.5, added with injectable water to the constant volume of 1000 ml, and homogeneously stirred and homogenized with high pressure homogenizer for the second time. The average diameter was detected as less than 100 nm. The emulsion was filled under the protection of nitrogen, and the container was sealed.

**EXAMPLE 18**

| | |
|---|---|
| vindesine sulfate | 0.5% |
| sesame oil | 15% |
| soybean lecithin | 10% |
| poloxamer188 | 2% |
| glycerin | 2.25% |
| sodium oleate | 0.1% |
| vitamin E | 0.05% |
| sodium sulfite | 0.1 % |
| injectable water | up to 100% |

Under the protection of inert gas atmosphere, injectable sesame oil 150g and vitamin E 0.5g were mixed homogeneously to obtain the oil phase. Soybean lecithin 100g, sodium oleate 1g, glycerin 22.5g, anhydrous sodium sulfite 1g and poloxamer188 20g were added into 600 ml injectable water and homogeneously stirred to obtain the aqueous phase. Under high-speed stirring, the oil phase and the aqueous phase were homogeneously mixed, and homogenized with high pressure homogenizer to obtain a coarse emulsion. The resulting emulsion was added with vindesine sulfate 5g, adjusted pH value to 9.0, added with injectable water to the constant volume of 1000 ml, homogeneously stirred and homogenized with high pressure homogenizer for the second time. The average diameter was detected as less than 100 nm. The emulsion was filled under the protection of nitrogen, and the container was sealed.

Taking the product of example 1 as example to study the physicochemical characteristics of the product obtained according to this invention:

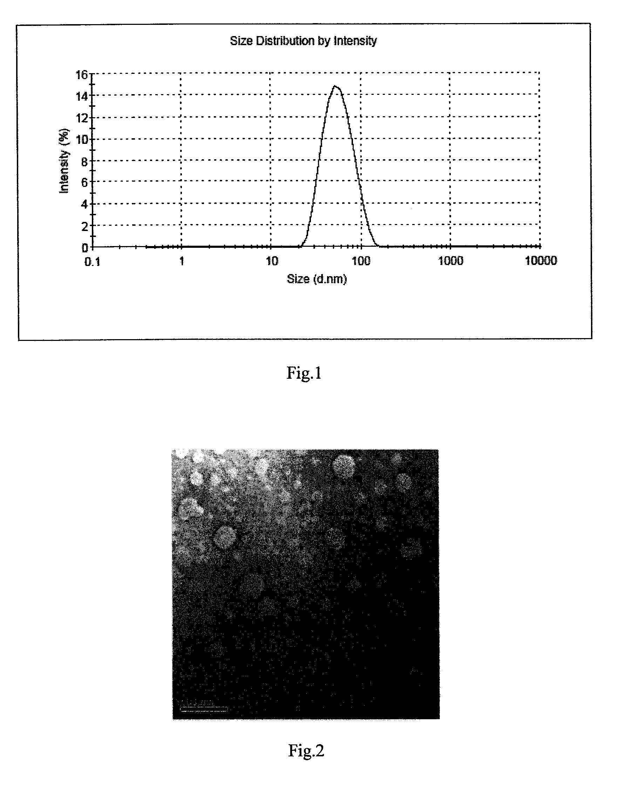

**Morphology:** Appropriate amount of the sample was diluted, dyed with phosphotungstic acid, and observed with transmission electron microscope (TEM). The result was shown in figure 2. The appearance of vinorelbine tartrate nano-emulsion was sphere or near to sphere.

**Compatiblity** stability: To satisfy the clinical medicament need, the present product was usually diluted with sodium chloride injection (0.9%) and glucose injection (5%). The present product was diluted with each of the diluents mentioned above to 5 and 10-fold solution, the particle size of these samples were determined respectively at 0, 1, 2, 4 and 6 hours. The results showed that there's no obvious change occurred in the particle size of the present product after diluting with sodium chloride injection (0.9%) and glucose injection (5%) within the 6 hours.

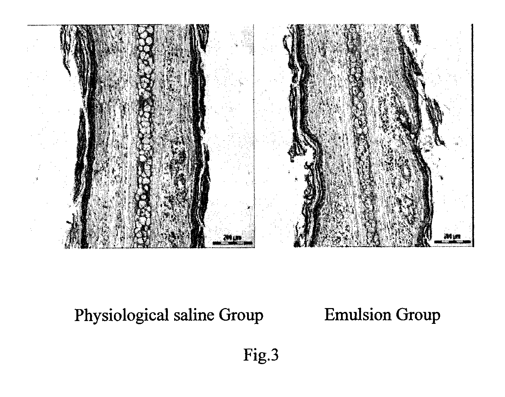

**Stimulation** test: Three healthy rabbits were tested. The rabbit was bundled, and the injection area was disinfected with 75% alcohol, slowly injected by syringe pumps with constant speed in ear edge vein with nano-emulsion injection to left ear and 0.9% sodium chloride injection to right ear as the negative control. Repeat the above-mentioned injection for three times every other day. The stimulation to rabbit vein after dosing, and symptoms such as blood stasis, dropsy or tissue necroswas etc were observed. The rabbit after 24 hours of the last administration were killed. The tissue of injection site was fixed with 10% formaldehyde. After routine paraffin-embedded, section, hematoxylin-eosin staining, the sample was observed by microscope. There was no obvious change in histopathology test after injection of vinorelbine nano-emulsion injection for three times. The results showed that intravenous injection of the present emulsion do not cause obvious stimulation (see figure3).

**Hemolytic** test: One rabbit was tested. About 20 ml blood was taken at the heart and removed into a triangle flask. Fibrin was wiped off from the blood by stirring with a bamboo stick, then the blood was transferred into a graduated centrifuge tube, and the tube was added with normal saline about 5 ml. After mixing solution homogeneously, the tube was centrifuged at 2500 r/min for 5 min, and then supernatant was discarded. The procedures of adding normal saline, mixing, centrifuging and discarding were repeated for several times until the supernatant appeared colorless transparent, and the red blood cells were obtained. The red blood cells were diluted to be 2% suspension (2%RBC) with normal saline by volume. Each solution was added into seven tubes orderly as listed in table 5. After the solutions were shaken softly, the tubes were kept warm for 4 hours in 37°C water bath. The following results were observed and recorded.

Table 5 Hemolytic test and results for product of example 1

| Tube number<br>solution(ml) | 1 | 2 | 3 | 4 | 5 | 6 | 7 |
|---|---|---|---|---|---|---|---|
| vinorelbine nano-emulsion injection | 0.1 | 0.2 | 0.3 | 0.4 | 0.5 | 0 | 0 |
| 0.9% sodium chloride injection | 2.4 | 2.3 | 2.2 | 2.1 | 2.0 | 2.5 | 0 |
| Distilled water | 0 | 0 | 0 | 0 | 0 | 0 | 2.5 |
| 2%RBC | 2.5 | 2.5 | 2.5 | 2.5 | 2.5 | 2.5 | 2.5 |
| 15 minutes | — | — | — | — | — | — | — |
| 30 minutes | — | — | — | — | — | — | — |
| 45 minutes | — | — | — | — | — | — | + |
| 1 hour | — | — | — | — | — | — | + |
| 2 hours | — | — | — | — | — | — | + |
| 3 hours | — | — | — | — | — | — | + |
| 4 hours | — | — | — | — | — | — | + |

Note: "—" means no hemolysis; "+"means whole hemolysis

Conclusion: The vinorelbine nano-emulsion had no obvious effect on hemolysis and agglutination to red cells of rabbit.


**Claims**

1.    A nano-emulsion injection of vinca alkaloids, **characterized in that** the said injection is an oil-in-water emulsion injection comprising vinca alkaloids or salts thereof, injectable oil, surfactants and injectable water, wherein the average diameter of the droplets of the emulsion is less than 100nm, and the pH of the emulsion is 7-9.

2.    The nano-emulsion injection according to claim 1, **characterized in that** the said emulsion does not comprise ingredients which can enhance the lipophilicity of vinca alkaloids in oil phase.

3.    The nano-emulsion injection according to claim 1, **characterized in that** the said vinca alkaloids are extractive, synthetic or semisynthetic, and the said salts are prepared by the reaction between vinca alkaloids and pharmaceutically acceptable acid.

4.    The nano-emulsion injection according to claim 3, **characterized in that** the said vinca alkaloids are selected from the group consisting of vinorelbine, vinblastine, vincristine, vindesine and vinrosidine, preferablely vinorelbine; the said salts are selected from the group consisting of tartrate, maleate, lactate, sulfate, malate, hydrochloride or phosphate, preferably tartrate.

5.    The nano-emulsion injection according to claim 1, **characterized in that** the said injectable oil is selected from the group consisting of one or more mineral oil, plant oil, animal oil and synthetic oil.

6. The nano-emulsion injection according to claim 5, **characterized in that** the said plant oil is selected from the group consisting of soybean oil, safflower oil, corn oil, coconut oil, castor oil, brucea javanica oil, palm oil, medium chain fatty acid triglycerides, peanut oil, cottonseed oil and a mixture thereof, preferably soybean oil, medium chain fatty acid triglycerides and a mixture thereof; the said animal oil is selected from the group consisting of fish oil, sperm oil and a mixture thereof.

7. The nano-emulsion injection according to claim 1, **characterized in that** the said surfactants are selected from the group consisting of phospholipids, nonionic surfactant and a mixture thereof.

8. The nano-emulsion injection according to claim 7, **characterized in that** the said phospholipids are selected from the group consisting of lecithin, soybean lecithin and a mixture thereof, preferablely egg lecithin; the said nonionic surfactant is selected from the group consisting of polyoxyethylene nonionic surfactant and polyethylene glycol nonionic surfactant.

9. The nano-emulsion injection according to claim 8, **characterized in that** the said polyoxyethylene nonionic surfactant is selected from the group consisting of tween 20, tween 40, tween 60, tween 80, tween 85, polyoxyethylene castor oil, poly (ethylene oxide) hydrogen castor oil, poloxamer 188 and a mixture thereof; the said polyethylene glycol nonionic surfactant is selected from the group consisting of polyethylene glycol stearate 15, polyethylene glycol-vitamin E succinate and a mixture thereof; poloxamer188 or polyethylene glycol stearate 15 are preferable.

10. The nano-emulsion injection according to any one of claims 1 to 9, **characterized in that**:

   the said vinca alkaloids are presented in the said nano-emulsion injection in the range of 0.05-5 w/v %, preferably 0.05-1.0 w/v %;
   the said injectable oil is presented in the said nano-emulsion injection ranges in the range of 2-30 w/v %, preferably 5-20 w/v %;
   the said surfactant is presented in the said nano-emulsion injection in the range of 1-20 w/v % , preferably 2-10 w/v %.

11. The nano-emulsion injection according to any one of claims 1 to 10, **characterized in that** the said nano-emulsion injection comprising:

   0.05-1.0 w/v % of vinorelbine or the tartrate form thereof based upon the said nano-emulsion injection;
   5-20 w/v % of soybean oil, medium chain fatty acid triglycerides or a mixture thereof based upon the said nano-emulsion injection;
   2-10 w/v % of combined surfactants based upon the said nano-emulsion injection, wherein the said combined surfactants are lecithin and poloxamer188, or lecithin and polyethylene glycol stearate 15; more preferably egg lecithin and poloxamer188, or egg lecithin and polyethylene glycol stearate 15.

12. The nano-emulsion injection according to any one of claims 1 to 11, **characterized in that** the said injection further comprises a metal chelator, wherein the said metal chelator is selected from the group consisting of EDTA, EDTA disodium salt, EDTA dicalcium salt and a mixture thereof; the said metal chelator is presented in the said nano-emulsion injection in the range of 0-0.5 w/v %.

13. The nano-emulsion injection according to any one of claims 1 to 12, **characterized in that** the said injection further comprises an antioxidant selected from the group consisting of water-soluble antioxidant and oil-soluble antioxidant, wherein the said water-soluble antioxidant is selected from the group consisting of sodium sulfite, sodium hydrogensulfite, sodium metabisulfite, ascorbic acid, sodium ascorbate, L-cysteine and a mixture thereof; the said oil-soluble antioxidant is vitamin E; the said antioxidant is presented in the said nano-emulsion injection in the range of 0-1 w/v %.

14. The nano-emulsion injection according to any one of claims 1 to 13, **characterized in that** the said injection further comprises an osmotic pressure regulator selected from the group consisting of glycerin, sorbitol, mannitol, glucose, sodium chloride and a mixture thereof; wherein the osmotic pressure regulator is presented in the said nano-emulsion injection in the range of 0-5 w/v %.

15. The nano-emulsion injection according to any one of claims 1 to 14, **characterized in that** the said injection further comprises a cosurfactant selected from the group consisting of oleic acid, sodium oleate, cholic acid, sodium cholate, deoxycholic acid, deoxysodium cholate and a mixture thereof; wherein the said cosurfactant is presented in the said nano-emulsion injection in the range of 0-1.5 w/v %.

16. A preparation process of the nano-emulsion injection according to any one of claims 1 to 15, comprising the following steps of:

preparing the oil phase and the aqueous phase respectively;
homogeneously mixing the oil phase and the aqueous phase with high speed to obtain coarse emulsion;
adding vinca alkaloids or the salts thereof into the coarse emulsion;
adjusting the pH value to 7-9, further adding water to the constant volume, then homogenizing the emulsion with high pressure homogenizer till the average diameter of the droplets being less than 100 nm.

17. A preparation process of the nano-emulsion injection according to any one of claims 1 to 15, comprising the following steps of:

preparing the oil phase and the aqueous phase respectively,
homogeneously mixing the oil phase and the aqueous phase , and homogenizing the emulsion with high pressure homogenizer to obtain a blank emulsion with an average diameter less than 100nm;
adding vinca alkaloids or the salts thereof into the blank emulsion;
adjusting the pH value to 7-9, stirring the blank emulsion thoroughly, further adding water to the constant volume.

Fig.1

Fig.2

Physiological saline Group          Emulsion Group

Fig.3

# INTERNATIONAL SEARCH REPORT

| International application No. |
| --- |
| PCT/CN2009/075409 |

**A. CLASSIFICATION OF SUBJECT MATTER**

See the extra sheet.
According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

DATABASE: WPI,EPODOC,CNPAT,CNKI,CA,MEDLINE,EMBASE

SEARCH TERMS: vinca, nano, emulsion, surface active agent, surfactant, vinorelbine, vinblastine, vincristine, vindesine, eldisine, inrosidine, leurocine, vinrosidine, phosphatide, phospholipid, phospholipins, polyoxyehtylene, polyethylene, glycol, macrogol, tween, poloxamer, chelator, chelating agent

**C. DOCUMENTS CONSIDERED TO BE RELEVANT**

| Category* | Citation of document, with indication, where appropriate, of the relevant passages | Relevant to claim No. |
| --- | --- | --- |
| A | CN1634058A(LI Xiaoxiang), 06 Jun.2005(06.07.2005), see the whole document | 1-17 |
| A | CN1859898A(SD PHARM INC et al.), 08 Nov. 2006(08.11.2006), see example 4 | 1-17 |
| A | CN101103962A(UNIV TONGJI MEDICAL COLLEGE MIDDLE CHINA SCI&TECHNOLOG), 16 Jan. 2008(16.01.2008) , see the whole document | 1-17 |
| A | CN1568938A(FAN Minhua), 26 Jan. 2005(26.01.2005) , see the whole document | 1-17 |
| A | CN1679576A(JIANGSU ZHENGDA TIANQING PHARM CO LT), 12 Oct. 2005(12.10.2005) , see the whole document | 1-17 |

☐ Further documents are listed in the continuation of Box C.　　☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 01 Mar. 2010(01.03.2010) | **18 Mar. 2010 (18.03.2010)** |

| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>**XU Lei**<br>Telephone No. (86-10)62411987 |
| --- | --- |

Form PCT/ISA /210 (second sheet) (July 2009)

## INTERNATIONAL SEARCH REPORT
### Information on patent family members

| International application No. |
|---|
| PCT/CN2009/075409 |

| Patent Documents referred in the Report | Publication Date | Patent Family | Publication Date |
|---|---|---|---|
| CN1634058A | 06.07.2005 | None | |
| CN1859898A | 08.11.2006 | US2006008480A1 | 12.01.2006 |
| | | WO2006017246A2 | 16.02.2006 |
| | | EP1773294A2 | 18.04.2007 |
| | | NO20070785A | 02.04.2007 |
| | | KR20070032816A | 22.03.2007 |
| | | AU2005271873A1 | 16.02.2006 |
| | | INKOLNP200700426E | 06.07.2007 |
| | | MXPA07000416A | 01.04.2007 |
| | | JP2008505972T | 28.02.2008 |
| | | BRPI0513178A | 29.04.2008 |
| | | TW200607525A | 01.03.2006 |
| | | CA2578574A | 16.02.2006 |
| | | ECSP077238A | 26.04.2007 |
| | | EA200700312A | 31.08.2007 |
| CN101103962A | 16.01.2008 | None | |
| CN1568938A | 26.01.2005 | CN100339067C | 26.09.2007 |
| CN1679576A | 12.10.2005 | CN100462076C | 18.02.2009 |

Form PCT/ISA /210 (patent family annex) (July 2009)

## INTERNATIONAL SEARCH REPORT

International application No.

PCT/CN2009/075409

CONTINUATION OF CLASSIFICATION OF SUBJECT MATTER:

A61K9/107(2006.01)i

A61K31/475(2006.01)i

A61P35/00(2006.01)i

Form PCT/ISA /210 (extra sheet) (July 2009)

**REFERENCES CITED IN THE DESCRIPTION**

**Patent documents cited in the description**

- CN 1859898 A
- CN 1771954 A
- CN 1679576 A
- CN 1634058 A